# FASCICULE DE BREVET EUROPEEN

(11) **EP 2 830 774 B1**
(45) Date de publication et mention de la délivrance du brevet: **02.01.2019**
(21) Numéro de dépôt: 13722542.1
(22) Date de dépôt: 25.03.2013
(51) Int. Cl.: B03B 9/06

(54) **PROCÉDÉ DE TRAITEMENT DE DÉCHETS, EN PARTICULIER D'ORDURES MÉNAGÈRES, ET INSTALLATION POUR LA MISE EN OEUVRE DE CE PROCÉDÉ**
VERFAHREN ZUR BEHANDLUNG VON ABFÄLLEN, INSBESONDERE HAUSMÜLL, UND ANLAGE ZUR DURCHFÜHRUNG VON DIESEM VERFAHREN
PROCESS FOR THE TREATMENT OF WASTE MATERIAL, ESPECIALLY DOMESTIC REFUSE, AND INSTALLATION FOR THE IMPLEMENTATION OF SAID PROCESS

(30) Priorité: 27.03.2012 FR 1252707
(43) Date de publication de la demande: 04.02.2015
(73) Titulaire: SUEZ Groupe, 92040 Paris la Défense Cedex (FR)
(72) Inventeur: SOMMAIN, Arnaud, F-34110 Mireval (FR)
(74) Mandataire: IPAZ
(86) Numéro de dépôt international: PCT/IB2013/052359
(87) Numéro de publication internationale: WO 2013/144817

(56) Documents cités:
- DE-A1-102007 049 479
- DE-A1-102009 032 122
- FR-A1- 2 951 095
- US-A1- 2006 289 356

## Description

L'invention est relative à un procédé de traitement de déchets, en particulier d'ordures ménagères, contenant des matières organiques mélangées à des indésirables, notamment métaux, matières minérales, plastiques, verre, procédé du genre de ceux selon lesquels :
- les déchets sont soumis à un premier tri par criblage,
- la fraction de déchets traversant le criblage est soumise à un traitement de pré-fermentation dans un tube rotatif avec alimentation à une extrémité et extraction à l'autre extrémité,
- et la matière sortant du tube de traitement de pré-fermentation est soumise à un traitement de méthanisation dans un digesteur.

Les déchets ménagers issus de collectes non sélectives contiennent différentes catégories de matériaux tels que des déchets putrescibles (déchets alimentaires, déchets verts), des papiers, des cartons, du verre, des plastiques, des métaux ferreux ou non ferreux, des tissus, des textiles sanitaires, des produits toxiques (piles électriques, pots de peinture...).

La méthanisation et le compostage de ces déchets ménagers en vue de les transformer en biogaz et en compost valorisable comporte généralement quatre étapes principales :
- une préparation mécanique des déchets qui vise à séparer les matières organiques biodégradables des autres fractions non valorisables en biogaz ou en compost ; un tube rotatif de pré-fermentation, généralement essentiellement horizontal, constitue un moyen pour une telle préparation;
- la méthanisation qui vise à produire une énergie renouvelable et est réalisée dans des enceintes horizontales ou verticales, agitées mécaniquement ou non ;
- le compostage, ou maturation aérobie, qui fait généralement intervenir une opération préalable de pressage du digestat provenant de la méthanisation, afin d'atteindre un niveau de teneur en matières sèches et une porosité permettant l'autocompostage du digestat, ou alors nécessite l'apport d'un agent structurant et son mélange avec le digestat pour obtenir un substrat compostable ;
- l'affinage final qui vise à retirer de façon complémentaire les contaminants restant après les deux opérations précédentes, et à préparer le compost à une granulométrie permettant sa valorisation agronomique.

La préparation mécanique peut comprendre un broyage en tête suivie d'étapes de tri granulométrique, comme enseigné par EP 0 131 319. Un tel procédé présente l'inconvénient majeur de fractionner les contaminants ou les matières valorisables (verre, plastique, tissu...), ce qui conduit généralement à des difficultés de tri et génère un risque fort de contamination du compost et nécessite des équipements de dimensions très importantes par rapport aux besoins.

FR 2 951 095 divulgue un procédé de traitement de déchets ménagers comportant plusieurs étapes de tri granulométrique et qui évite l'utilisation d'un tube rotatif de pré-fermentation qui mélange des déchets valorisables et des déchets non valorisables en compost.

Cependant, le tube rotatif de pré-fermentation suivi de tris granulométriques présente comme avantage une fragmentation des papiers et des cartons et un début de dégradation biologique permettant ensuite une séparation de la fonction méthanisable par tris granulométriques.

Le niveau de qualité des déchets en sortie du tube rotatif demande à être amélioré car n'étant généralement pas constant ni optimal. En effet, la maille de tamisage en sortie de tube est une constante : si la matière organique n'est pas suffisamment réduite, elle sera éliminée et conduira à une perte importante pour la méthanisation. A contrario, si des impuretés physiques passent au travers de la maille, ces impuretés se retrouveront dans le substrat introduit dans les digesteurs. De telles impuretés n'ont pas d'intérêt méthanogène et peuvent compromettre à la fois la sécurité et la pérennité du processus de méthanisation (accumulation de plastiques flottants, sédimentation du verre dans le fond des digesteurs...). Enfin, ces impuretés seront très difficilement extractibles du digestat en fin de maturation aérobie pour permettre une normalisation du compost produit.

L'invention a pour but, surtout d'obtenir une fraction organique homogène de qualité et la plus exempte possible d'indésirables, avec une production de biogaz optimale au regard du dimensionnement du digesteur utilisé. L'invention vise aussi à produire un digestat à forte teneur en matières sèches afin de pouvoir rendre facultatif son pressage avant maturation aérobie, sans pénaliser le digestat, et à fournir un compost conforme aux normes appliquées dans les pays concernés par le procédé.

Selon l'invention, le procédé de traitement de déchets, en particulier d'ordures ménagères, du genre défini précédemment, est caractérisé en ce que :
- les déchets tels que collectés, et sans broyage préalable, sont soumis au premier tri par criblage sur tamis ayant une dimension de maille L1;
- la fraction des déchets ayant traversé ce premier tri, qui comprend des éléments dont la grande dimension est inférieure à la valeur L1, est envoyée au traitement de pré-fermentation dans le tube rotatif;
- l'alimentation en déchets du tube rotatif est effectuée régulièrement dans le temps, en lissant les à-coups de collecte ;
- le temps de séjour des déchets dans le tube rotatif est sensiblement constant et au moins égal au temps nécessaire pour que la quasi-totalité de la matière organique se trouve dans une fraction granulométrique inférieure à celle des indésirables ;
- et les déchets après sortie du tube rotatif, et avant entrée dans le digesteur, sont soumis à un criblage par tamis présentant une dimension de maille L2 assurant une coupure entre les matières organiques de granulométrie plus fine, et les indésirables.

Le temps de séjour des déchets dans le tube rotatif est au moins égal au temps nécessaire pour qu'au moins 75% en masse de la matière organique en sortie de tube de pré-fermentation se trouve dans une fraction granulométrique inférieure à celle des indésirables.

Le procédé, par une alimentation permanente et régulière du tube rotatif, permet de lisser les apports de matière irréguliers dans le temps provoqués notamment par le fait que la collecte a généralement lieu cinq jours sur sept seulement, et de huit à douze heures par jour.

Avantageusement, l'alimentation en déchets du tube rotatif et l'extraction des déchets traités sont effectuées en continu, le débit d'extraction correspondant au débit d'alimentation, moins la perte matière et par évaporation, et étant sensiblement constant sur une durée de plusieurs heures, de préférence d'au moins 10 h et avantageusement de 24h.

En variante, l'alimentation en déchets du tube rotatif peut être effectuée par fournées, à intervalles de temps réguliers, relativement réduits, de préférence inférieurs à 14 heures, et la masse de déchets introduite dans le tube à chaque fournée de chargement est sensiblement la même.

La dimension L1 de la maille du premier tri peut être comprise entre 200 et 600 mm, de préférence entre 350 et 500 mm et avantageusement égale à 450 mm.

Le temps de séjour des déchets dans le tube rotatif peut être compris entre deux et quatre jours, de préférence entre deux jours et demi et trois jours et demi et avantageusement trois jours. La dimension L2 de la maille du tamis avant entrée dans le digesteur peut être comprise entre 5 et 14 mm, de préférence entre 7 et 12 mm avantageusement égale à 8 mm.

L'alimentation du tube rotatif peut mettre en oeuvre au moins une trémie doseuse propre à lisser les éventuels à-coups d'alimentation provenant de variations brutales de granulométrie et/ou de la masse volumique du flux de déchets collectés.

Plusieurs étages de tri avec dimensions de mailles plus petites à chaque étage suivant peuvent être prévus entre la sortie du tube rotatif et le dernier criblage par tamis présentant une dimension de maille L2.

L'installation peut comporter un crible à effet trampoline assurant le dernier criblage avant le digesteur.

De préférence, le digesteur est horizontal, agité mécaniquement, et des moyens sont prévus pour assurer une recirculation d'une fraction du digestat sortant à l'entrée du digesteur, avantageusement selon un taux de recirculation d'au moins 200%.

En aval du tube rotatif et en amont du dernier criblage, les déchets peuvent être soumis à un tri par rebond/adhérence.

Une trémie mélangeuse peut être prévue en aval du dernier criblage, et en amont du digesteur, et la fraction de digestat recirculée est introduite, pour une partie supérieure à 50%, dans la trémie mélangeuse, et pour l'autre partie à l'entrée du digesteur, en aval de la trémie mélangeuse.

L'invention est également relative à une installation de traitement de déchets pour la mise en oeuvre du procédé défini précédemment, cette installation comprenant :
- un premier poste de tri par criblage,
- un tube rotatif de traitement de pré-fermentation de la fraction des déchets ayant traversé ce premier tri,
- et un digesteur pour soumettre à un traitement de méthanisation la matière sortant du tube de traitement de pré-fermentation,
et étant caractérisée en ce que :
- le premier poste de tri par criblage présente une dimension de maille L1 pour les déchets tels que collectés, et sans broyage préalable;
- des moyens d'alimentation en déchets du tube rotatif sont prévus pour assurer une alimentation de manière régulière, sans interruption dans le temps, en lissant les à-coups de collecte ;
- le tube rotatif est prévu pour que le temps de séjour des déchets soit au moins égal au temps nécessaire pour que la quasi-totalité de la matière organique se trouve dans une fraction granulométrique inférieure à celle des indésirables ;
- et, en amont du digesteur, un poste de criblage par tamis présentant une dimension de maille L2 est prévu pour assurer une coupure entre les matières organiques de granulométrie plus fine, et les indésirables.

L'installation comporte avantageusement plusieurs tubes rotatifs travaillant en parallèle, en particulier trois tubes rotatifs.

La dimension L1 de la maille du tamis du premier tri peut être comprise entre 200 et 600 mm, de préférence entre 350 et 500 mm et avantageusement égale à 450 mm.

La dimension L2 de la maille du tamis avant entrée dans le digesteur peut être comprise entre 5 et 14 mm, de preference entre 7 et 12 mm avantageusement égale à 8 mm.

Les moyens d'alimentation du tube rotatif peuvent comprendre au moins une trémie doseuse propre à lisser les éventuels à-coups d'alimentation provenant de variations brutales de granulométrie et/ou de la masse volumique du flux de déchets collectés.

Plusieurs étages de tri, avec dimensions de mailles plus petites à chaque étage suivant, peuvent être prévus entre la sortie du tube rotatif et le dernier criblage par tamis présentant une dimension de maille L2.

L'installation peut comporter un crible à effet trampoline assurant le dernier criblage avant le digesteur.

Avantageusement, le digesteur est horizontal, agité mécaniquement, et des moyens sont prévus pour assurer une recirculation d'une fraction du digestat sortant à l'entrée du digesteur.

Une trémie mélangeuse peut être prévue en aval du dernier criblage, et en amont du digesteur, et la fraction de digestat recirculée est introduite, pour une partie supérieure à 50%, dans la trémie mélangeuse, et pour l'autre partie à l'entrée du digesteur, en aval de la trémie mélangeuse.

L'invention consiste, mises à part les dispositions exposées ci-dessus, en un certain nombre d'autres dispositions dont il sera plus explicitement question ci-après à propos d'un exemple de réalisation décrit avec référence aux dessins annexés, mais qui n'est nullement limitatif. Sur ces dessins :
Fig. 1 est un schéma d'un procédé de traitement selon l'invention, et
Fig. 2 est un diagramme illustrant schématiquement l'évolution de la granulométrie des matières organiques et des matières organiques synthétiques ou inorganiques de mêmes dimensions dans un tube rotatif de traitement de pré-fermentation, en fonction du temps de séjour porté en abscisse, tandis que le diamètre moyen de la granulométrie est porté en ordonnée.

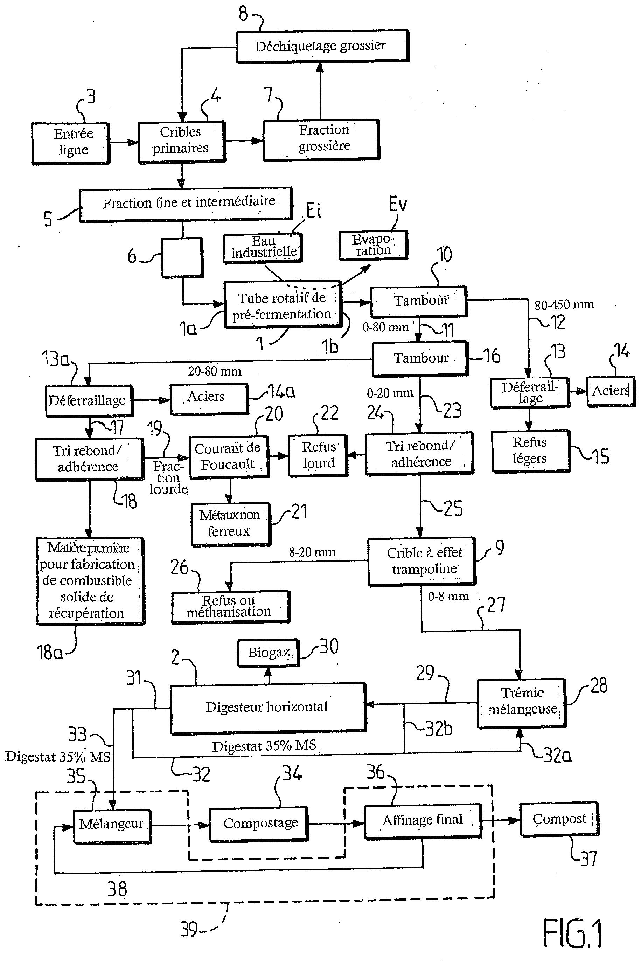

Un objectif de l'invention est d'obtenir :
- une qualité de fraction organique homogène, après passage dans un tube rotatif de traitement de pré-fermentation 1 (Fig. 1) et la plus exempte possible d'indésirables après criblage,
- et une production de biogaz optimale au regard du dimensionnement du digesteur 2 utilisé.

L'invention vise aussi à fournir un digestat à forte teneur en matières sèches, de sorte de pouvoir rendre facultatif son pressage avant maturation aérobie sans pénaliser ce digestat, et un compost conforme aux normes appliquées dans les pays concernés par le procédé.

Le terme « digestat » désigne le produit sortant du digesteur, constitué par la matière organique après digestion anaérobie,

Il est apparu que le niveau de qualité des matières en sortie du tube rotatif de pré-fermentation n'était pas constant. Il a pu être établi que les apports de matières irréguliers dans le temps, notamment cinq jours sur sept seulement, et huit à douze heures par jour, entraînaient un temps de séjour variable des matières dans le tube rotatif et empêchaient d'atteindre le niveau de qualité souhaité.

La maille de tamisage en sortie du tube 1 est une constante ; si la matière organique n'est pas suffisamment réduite, elle sera éliminée et conduira à une perte importante pour la méthanisation. A contrario, si les impuretés physiques, constituant les indésirables, passent au travers de cette maille, ces impuretés se retrouveront dans le substrat introduit dans le digesteur, ce qui est à éviter.

Selon la présente description, la dimension d'une maille de tamis correspond, pour une maille carrée ou rectangulaire à la longueur de la diagonale de la maille, et pour une maille circulaire, au diamètre de cette maille.

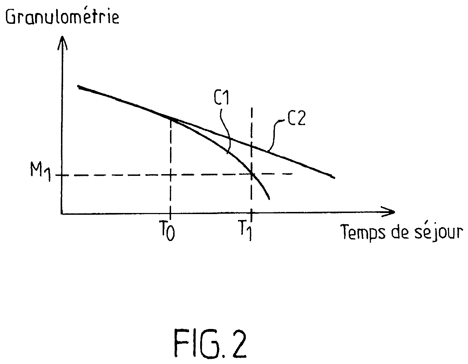

Il a pu être établi que la granulométrie des matières organiques et des indésirables évoluait différemment en fonction du temps de séjour dans le tube rotatif. Sur Fig. 2 on a illustré schématiquement la variation de la granulométrie portée en ordonnée, sous forme d'une dimension de maille à travers laquelle peut passer le produit en sortie du tube 1, en fonction du temps de séjour du produit dans le tube 1, porté en abscisse.

C1 correspond à la courbe de granulométrie des matières organiques tandis que C2 correspond à la courbe de granulométrie des matières organiques synthétiques, inorganiques ou minérales, indésirables. Au-delà d'un temps de séjour T₀, la courbe C1 se détache vers le bas par rapport à la courbe C2. Après un temps de séjour T₁, la granulométrie des matières organiques (courbe C1) est nettement inférieure à celle des matières inorganiques ou organiques synthétiques. En choisissant un tamis dont la maille présente une dimension M1 correspondant à la granulométrie de la courbe C1, ou qui est légèrement supérieure, notamment de 1 mm, à cette granulométrie, on assure une séparation efficace entre les matières organiques et les indésirables, avec une perte minimale de matières organiques.

A titre d'exemple non limitatif, T1 peut être compris entre 2 et 3,5 jours , et M1 peut être compris entre 7 et 9mm, avantageusement égal à 8mm.

Fig. 1 des dessins illustre un schéma d'ensemble d'une ligne de traitement mettant en oeuvre le procédé de l'invention.

Les déchets collectés, notamment par camions-bennes, sont introduits dans une entrée 3 de ligne de traitement et sont envoyés sur un ou des cribles primaires 4, notamment à l'aide de transporteurs à bande ou à tabliers métalliques. Chaque crible 4 est constitué par un tamis dont les mailles ont une dimension L1 prévue pour retenir les fractions grossières dont la grande dimension est supérieure à L1. La dimension L1 est avantageusement comprise entre 400 et 500 mm, de préférence égale à 450 mm. La fraction 5 traversant les cribles 4 constitue une fraction fine et intermédiaire qui sert à alimenter le tube rotatif 1, essentiellement horizontal.

Les mêmes quantités de déchets sont introduites, à débit constant, dans le tube 1 et extraites en continu, en quasi-permanence. L'alimentation du tube 1 est optimisée par utilisation de trémies 6, en particulier de trémies doseuses (trémie de dosage dont le fond est équipé de lattes (ou équivalent) à déplacements séquentiels permettant l'avancement du produit), qui permettent de lisser les éventuels à-coups d'alimentation provenant de variations brutales de granulométrie et/ou de la masse volumique du flux de déchets. Selon le schéma, la trémie 6 de lissage est installée en aval de la sortie de la fraction 5 et en amont du tube 1. En variante, la trémie de lissage 6 pourrait être située entre l'entrée 3 de ligne et les cribles primaires 4. Dans ce dernier cas, l'équipement ne satisfait pas aussi bien à la limitation des à-coups (la rupture de charge permise par la trémie en amont des tubes, selon le schéma de Fig.1, permet des actions de maintenance, un traitement discontinu des ordures).

La fraction la plus grossière 7 de dimension supérieure à celle des mailles des cribles 4, par exemple supérieure à 450 mm, et qui n'a pas traversé les cribles, peut être soumise à un déchiquetage grossier 8 avant d'être réintroduite dans les tubes de pré-fermentation 1 ou en variante dans les cribles primaires 4, ou d'être traitée séparément.

De l'eau industrielle Ei est introduite dans les tubes de préfermentation 1 pour favoriser l'activité biologique, L'échauffement des réactions biologiques provoque l'évaporation Ev de tout ou partie de cette eau industrielle.

Le tube rotatif 1 est horizontal et présente une paroi cylindrique fermée dont la surface intérieure est munie de profilés, par exemple profilés en U tournant leur concavité radialement vers l'intérieur, régulièrement espacés, s'étendant suivant des génératrices du cylindre, et propres à entraîner en rotation des déchets introduits à l'extrémité 1a. Les déchets progressent dans le tube 1 sous l'effet de la poussée des matières chargées à l'entrée 1a, et sont extraits à la sortie 1b après un temps de séjour T dans le tube 1.

En assurant un temps de séjour T approprié des déchets dans le tube rotatif 1 (T est égal ou supérieur à T1), combiné avec une alimentation régulière, on obtient à la sortie 1b, un mélange dans lequel les matières organiques ont une granulométrie égale ou inférieure à M1, en particulier inférieure à 8 mm, tandis que les indésirables ont une granulométrie supérieure. La séparation des matières organiques à introduire à l'entrée 2a du digesteur 2, relativement aux indésirables, est assurée par un crible 9 à mailles dont la dimension correspond à celle de la granulométrie des matières organiques, notamment 8 mm. Le crible 9 est avantageusement un crible à effet trampoline ; un tel crible est prévu pour assurer un tamisage sur une toile élastique successivement tendue et relâchée, offrant une légère déformation des mailles qui se décolmatent, ce système permettant un tamisage de produit humide dans de très petites granulométries.

Plusieurs tubes rotatifs 1 de pré-fermentation peuvent être prévus pour travailler en parallèle, notamment trois tubes de pré-fermentation. La régularité de l'alimentation et de l'extraction des tubes permet d'établir un produit fini de composition homogène et de profil granulométrique constant au niveau de la fraction 27 à introduire dans le digesteur 2. Le taux d'indésirables (plastiques, verre, cailloux, métaux) est pratiquement nul dans la fraction de granulométrie inférieure à M1.

Pour éviter de surcharger le crible 9 avec des indésirables dont les dimensions sont nettement supérieures à la maille du crible 9, plusieurs étages de tri sont prévus en amont du crible 9.

Un premier étage de tri à la sortie 1b du tube 1 est assuré par un tamis rotatif 10, ou tambour perforé, qui laisse passer une fraction 11, par exemple de granulométrie 0-80 mm, et rejette un refus 12 correspondant par exemple à une granulométrie 80-450 mm. Ce refus 12 est soumis à une étape 13 de déferraillage permettant de séparer les aciers 14 des refus légers 15 à haut pouvoir calorifique inférieur PCI.

La fraction 11 qui a traversé le tamis 10 est introduite dans un second tamis rotatif 16 à mailles de dimension inférieure à celle du tamis 10, notamment à mailles de 20 mm. Le refus du tamis 16, qui correspond dans l'exemple considéré à une granulométrie de 20-80mm, est soumis à une étape de déferraillage 13a qui sépare les aciers 14a d'une autre fraction 17. Cette fraction 17 est soumise à une étape 18 de tri rebond/adhérence . Ce tri consiste à projeter la matière sur une plaque rigide ou un convoyeur à bande incliné, ce qui engendre le rebond du refus lourd et l'adhérence des parties plus légères, notamment des matières organiques. La fraction lourde 19 issue du tri 18 est soumise à une séparation par courants de Foucault 20 qui permet de séparer les métaux non ferreux 21 des refus lourds 22. La fraction plus légère 18a issue de l'étape 18 de tri rebond/adhérence constitue de la matière première pour la fabrication de combustible solide de récupération.

La fraction 23 qui a traversé les mailles du tamis 16, par exemple la fraction de granulométrie 0-20 mm, est soumise à un tri 24, de préférence du type rebond/adhérence qui sépare des refus lourds 22 d'une fraction plus légère 25 contenant les matières organiques.

Cette fraction 25 est ensuite soumise à une séparation par le crible 9 présentant des mailles de dimension égale ou inférieure à M1 correspondant à la coupure entre la granulométrie des matières organiques et celle des indésirables selon Fig. 2, après un temps de séjour au moins égal à T1 dans le tambour rotatif 1. Le crible 9 sépare un refus 26 dont la granulométrie est, par exemple, de 8-20 mm, et une fraction fine 27, dont la granulométrie est, par exemple, de 0-8 mm. Le crible 9 est avantageusement un crible à effet trampoline .

La fraction fine 27, sortant du crible 9, est introduite dans une trémie mélangeuse 28 donnant en sortie 29 un mélange homogène qui est introduit à l'entrée du digesteur 2 horizontal agité mécaniquement. Le digesteur 2 est constitué par un tube cylindrique d'axe horizontal dans lequel sont disposés des moyens d'agitation de la matière introduite. Les moyens d'agitation peuvent comprendre un arbre rotatif coaxial muni de bras radiaux espacés, pourvus de palettes de brassage. Le digesteur 2 produit du biogaz récupéré au niveau d'une évacuation 30 située en partie haute du digesteur. Le digestat est évacué par une sortie 31 opposée à l'entrée du digesteur. Une fraction 32 du digestat est recirculée selon une partie 32a dans la trémie mélangeuse 28 et, selon une autre partie 32b, à l'entrée du digesteur 2, en aval de la trémie 28.

Le digestat sert de vecteur pour le transport du substrat organique constitué par la fraction 27. Le taux de matières sèches du mélange est maîtrisable,d'autant que l'incorporation de liquide (eau de ville, eaux industrielles etc...) est également possible, et le mélange réalisé dans la trémie 28 est intime et homogène.

Le taux de recirculation R/Q du digestat est de préférence supérieur à 200 % de manière à assurer une matière infiniment mélangée. Le taux de recirculation R/Q correspond au rapport du débit massique R de la fraction 32 de digestat recirculée, au débit Q de la fraction 27 de matière introduite dans la trémie 28.

La fraction 32a recirculée dans la trémie 28 est de préférence supérieure à la fraction 32b recirculée à l'entrée du digesteur, en aval de la trémie 28. La fraction 32a est avantageusement au moins égale à 60 % en masse de la fraction recirculée 32.

A titre d'exemple numérique non limitatif, la trémie 28 peut avoir un volume de 2 m³ ; la fraction 27 introduite dans la trémie 28 peut être de 1200 t/mois, la fraction 32a de 1800 t/mois et la fraction 32b de 1740 t/mois. La fraction totale 32 recirculée est de 3540 t/mois. Selon cet exemple, le taux de recirculation est de 3540/1200, soit environ 300 %.

Selon l'invention, la composition, ou recette, du mélange introduit dans le digesteur 2 est améliorée par la recirculation du digestat qui se substitue à un fluide de mélange tel que de l'eau industrielle. Le pouvoir mouillant du digestat est supérieur à celui du liquide. Il est ainsi possible d'augmenter significativement la charge organique dans le mélange tout en réduisant le volume global.

La recirculation du digestat dans la trémie mélangeuse 28 augmente la capacité de traitement du digesteur d'environ 30 %. En effet, le volume nécessaire pour introduire une tonne de substrat organique, provenant de la fraction 27, est considérablement réduit. A titre d'exemple non limitatif, la capacité d'introduction en substrat organique est passée d'environ 25 t/jour à 40 t/jour selon l'invention.

A viscosité identique, le taux de matières sèches du mélange 29 issu de la nouvelle composition est plus élevé. Il est ainsi possible d'augmenter significativement la charge organique dans le mélange tout en réduisant le volume global réduit.

Le digestat présente un taux de matières sèches d'environ 35 % pouvant aller jusqu'à 40 %, satisfaisant pour permettre le post-traitement par compostage ; cette siccité simplifie très largement l'exploitation et améliore les rendements. La bonne gestion de la viscosité de la liqueur entrant dans le digesteur permet de satisfaire les conditions mécaniques de brassage dans le digesteur, sans contraintes excessives.

La fraction 33 du digestat, qui n'est pas recirculée, est utilisée pour l'étape 34 de compostage. Pour cela, la fraction 33 est introduite dans un mélangeur 35 dont la sortie est envoyée au compostage 34. La matière en sortie du compostage 34 est envoyée à une étape 36 d'affinage final qui fournit, en sortie, le compost 37. Une fraction 38 de l'affinage est avantageusement recirculée à l'entrée du mélangeur 35.

Le mélangeur 35 et l'affinage 36 constituent un traitement 39 de structuration du compost, schématisé par un contour en tirets. Ce traitement pourrait être remplacé par une étape de déshydratation, en sortie du compostage 34. La fraction 33 serait alors directement admise à l'entrée du compostage 34.

L'invention permet d'obtenir une qualité de fraction organique 29, envoyée au digesteur, qui est homogène et exempte le plus possible d'indésirables, ce qui est très favorable à la stabilité de la méthanisation.

La faible granulométrie de la fraction organique 0-8 mm augmente la surface de contact entre les bactéries et le substrat, ce qui est favorable également à la méthanisation. Le taux réduit d'indésirables et l'homogénéité constante du produit permettent de garantir la conformité de tous les lots de fabrication de compost. Cette faible granulométrie est exploitée au mieux dans le digesteur horizontal 2 dont l'agitation mécanique permet le dégazage du biogaz produit dans la liqueur.

La production de biogaz par le digesteur 2 est optimale au regard du dimensionnement du digesteur utilisé et permet d'obtenir un digestat à forte teneur en matières sèches, d'au moins 30 % MS jusqu'à 38% de MS, ce qui permet de rendre facultatif un pressage mécanique avant maturation aérobie, sans pénaliser le digestat, et d'obtenir un compost conforme aux normes.

Grâce au procédé de séparation mécano-biologique faisant intervenir le tube rotatif 1, les étapes de traitement du produit en entrée et sortie du tube par criblage permettent d'optimiser la qualité de chacune des catégories de matériaux contenus dans les déchets par rapport à sa voie de valorisation :
- les matières organiques les plus instables, très réactives, sont compostées rapidement dans le tube 1 ;
- le substrat organique (fraction 27) destiné à la méthanisation est optimisé;
- la séparation des métaux ferreux et non-ferreux de grande propreté est optimale ;
- les flux à fort potentiel calorifique sont bien séparés ;
- la propreté du compost final est assurée.

L'amélioration de la stabilité du digesteur 2 obtenue selon l'invention a un impact sur le rendement de production électrique à la tonne de substrat organique introduit dans le digesteur. Selon un exemple, le rendement moyen a connu une augmentation d'environ 10 %.

Le procédé de l'invention est utilisable industriellement pour dimensionner une unité de traitement de déchets avec méthanisation, en maximisant à la fois :
- le taux d'extraction de la matière organique non synthétique présente initialement dans les déchets, grâce à une alimentation continue du tube rotatif 1 et un temps de séjour optimal dans ce tube, tout en améliorant la propreté de celle-ci ;
- la production de biogaz du procédé de méthanisation utilisant un digesteur horizontal et le conduisant en mode assimilé à un mode infiniment mélangé, en raison du taux de recirculation, au lieu du mode piston habituel, et en maintenant la liqueur présente dans le digesteur à une teneur en matières sèches optimale voisine de 35 %.

## Revendications

1. Procédé de traitement de déchets, en particulier d'ordures ménagères, contenant des matières organiques mélangées à des indésirables, notamment métaux, matières minérales, plastiques, verre, procédé selon lequel :
- les déchets sont soumis à un premier tri par criblage (4),
- la fraction de déchets traversant le criblage est soumise à un traitement de pré-fermentation dans un tube rotatif (1) avec alimentation à une extrémité et extraction à l'autre extrémité,
- et la matière sortant du tube de traitement de pré-fermentation est soumise à un traitement de méthanisation dans un digesteur (2),
**caractérisé en ce que** :
- les déchets tels que collectés, et sans broyage préalable, sont soumis au premier tri (4) par criblage sur tamis ayant une dimension de maille L1;
- la fraction des déchets ayant traversé ce premier tri, qui comprend des éléments dont la grande dimension est inférieure à la valeur L1, est envoyée au traitement de pré-fermentation dans le tube rotatif (1);
- l'alimentation en déchets du tube rotatif est effectuée régulièrement dans le temps, en lissant les à-coups de collecte ;
- le temps de séjour (T) des déchets dans le tube rotatif est sensiblement constant et au moins égal au temps nécessaire pour que la quasi-totalité de la matière organique se trouve dans une fraction granulométrique inférieure à celle des indésirables ;
- et les déchets après sortie du tube rotatif, et avant entrée dans le digesteur, sont soumis à un criblage (9) par tamis présentant une dimension de maille L2 assurant une coupure entre les matières organiques de granulométrie plus fine, et les indésirables.

2. Procédé selon la revendication 1, **caractérisé en ce que** le temps de séjour (T) des déchets dans le tube rotatif (1) est au moins égal au temps nécessaire pour qu'au moins 75 % en masse de la matière organique en sortie du tube de pré-fermentation se trouve dans une fraction granulométrique inférieure à celle des indésirables.

3. Procédé selon la revendication 1 ou 2, **caractérisé en ce que** l'alimentation en déchets du tube rotatif (1) et l'extraction des déchets traités sont effectuées en continu, le débit d'extraction correspondant au débit d'alimentation, moins la perte matière et par évaporation, et étant sensiblement constant sur une durée de plusieurs heures, de préférence d'au moins 10 h et avantageusement de 24h.

4. Procédé selon l'une quelconque des revendications précédentes, **caractérisé en ce que** la dimension L1 de la maille du premier tri est comprise entre 200 et 600 mm, de préférence entre 350 et 500 mm et avantageusement égale à 450 mm.

5. Procédé selon l'une quelconque des revendications précédentes, **caractérisé en ce que** le temps de séjour (T) des déchets dans le tube rotatif (1) est compris entre deux et quatre jours , de préférence entre deux jours et demi et trois jours et demi et avantageusement trois jours.

6. Procédé selon l'une quelconque des revendications précédentes, **caractérisé en ce que** la dimension (L2) de la maille du tamis avant entrée dans le digesteur est comprise entre 5 et 14 mm, de préférence entre 7 et 12 mm avantageusement égale à 8 mm.

7. Procédé selon l'une quelconque des revendications précédentes, **caractérisé en ce que** l'alimentation du tube rotatif met en oeuvre au moins une trémie doseuse (6) propre à lisser les éventuels à-coups d'alimentation provenant de variations brutales de granulométrie et/ou de la masse volumique du flux de déchets collectés.

8. Procédé selon l'une quelconque des revendications précédentes, **caractérisé en ce que** plusieurs étages de tri (10,16) avec dimensions de mailles plus petites à chaque étage suivant sont prévus entre la sortie du tube rotatif (1) et le dernier criblage (9) par tamis présentant une dimension de maille L2.

9. Procédé selon l'une quelconque des revendications précédentes, **caractérisé en ce que** le dernier criblage avant digesteur (2) est assuré par un crible à effet trampoline (9).

10. Procédé selon l'une quelconque des revendications précédentes, **caractérisé en ce que** le digesteur (2) est horizontal, agité mécaniquement, et une fraction du digestat sortant du digesteur est recirculée à l'entrée, de préférence selon un taux de recirculation d'au moins 200%.

11. Procédé selon la revendication 10, **caractérisé en ce qu'**une trémie mélangeuse (28) est prévue en aval du dernier criblage (9), et en amont du digesteur (2), et la fraction de digestat recirculée est introduite, pour une partie supérieure à 50%, dans la trémie mélangeuse (28), et pour l'autre partie à l'entrée du digesteur, en aval de la trémie mélangeuse.

12. Installation de traitement de déchets pour la mise en oeuvre d'un procédé selon l'une quelconque des revendications précédentes , comprenant :
- un premier poste de tri par criblage (4),
- un tube rotatif (1) de traitement de pré-fermentation de la fraction des déchets ayant traversé ce premier tri,
- et un digesteur (2) pour soumettre à un traitement de méthanisation la matière sortant du tube de traitement de pré-fermentation,
**caractérisée en ce que** :
- le premier poste de tri par criblage (4) présente une dimension de maille L1 pour les déchets tels que collectés, et sans broyage préalable;
- des moyens d'alimentation (6) en déchets du tube rotatif (1) comprennent au moins une trémie (6) doseuse propre à lisser les éventuels à-coups d'alimentation provenant de variations brutales de granulométrie et/ou de la masse volumique du flux de déchets collectés, pour assurer une alimentation de manière régulière, sans interruption dans le temps ;
- le tube rotatif (1) est prévu pour que le temps de séjour des déchets soit au moins égal au temps nécessaire pour que la quasi-totalité de la matière organique se trouve dans une fraction granulométrique inférieure à celle des indésirables ;
- et, en amont du digesteur, un poste de criblage (9) par tamis présentant une dimension de maille L2 est prévu pour assurer une coupure entre les matières organiques de granulométrie plus fine, et les indésirables.

13. Installation selon la revendication 12, **caractérisée en ce qu'**elle comporte plusieurs tubes rotatifs (1) travaillant en parallèle, en particulier trois tubes rotatifs.

14. Installation selon la revendication 12 ou 13, **caractérisée en ce que** la dimension L1 de la maille du tamis du premier tri est comprise entre 200 et 600 mm, de préférence entre 350 et 500 mm et avantageusement égale à 450 mm.

15. Installation selon l'une quelconque des revendications 12 à 14, **caractérisée en ce que** la dimension L2 de la maille du tamis avant entrée dans le digesteur (2) est comprise entre 5 et 14 mm, de préférence entre 7 et 12 mm, avantageusement égale à 8 mm.

16. Installation selon l'une quelconque des revendications 12 à 16, **caractérisée en ce qu'**elle comporte un crible à effet trampoline (9) assurant le dernier criblage avant le digesteur (2).

17. Installation selon l'une quelconque des revendications 12 à 17, **caractérisée en ce que** le digesteur (2) est horizontal, agité mécaniquement, et que des moyens sont prévus pour assurer une recirculation d'une fraction du digestat sortant à l'entrée du digesteur.

## Patentansprüche

1. Verfahren zur Abfallentsorgung, insbesondere von Haushaltsmüll, der ein Gemisch von organischen sowie von unerwünschten Stoffen wie Metalle, Mineralstoffe, Plastik und Glas enthält; wobei gemäß dem Verfahren:
- der Abfall einer ersten Aussortierung durch den Screening-Prozess (4) unterliegt;
- die Müllfraktion durch das Screening einer Vorbehandlung durch Fermentierung in einem Drehrohr (1) unterworfen ist; mit einer Zuführung an der Extremität sowie der Extraktion an der anderen Extremität,
- die Materie, die aus dem Bearbeitungsrohr, in dem die Vorbehandlung durch Fermentierung stattfindet, austritt, einer Methan-Behandlung in einem Gärbehälter (2) unterzogen wird;
**gekennzeichnet dadurch, dass**:
- die Abfälle im originellen Sammelzustand, ohne vorherige Zerkleinerung, einer ersten Triage durch den Screening-Prozess (4) auf einem Sieb mit Einheitszellengröße L1 unterworfen werden;
- die Fraktion der Abfallanteile, die die erste Triage durchlaufen und die Elemente enthalten, deren Dimension geringer als L1 ist, zur Vorbehandlung durch Fermentierung im Drehrohr (1) weitergeleitet werden;
- die Zufuhr des Abfalls in das Bearbeitungsrohr kontinuierlich während einer gewissen Zeitspanne durch reibungslose Glättung der Materie stattfindet;
- die Aufenthaltszeit (T) des Abfalls im Drehrohr im Wesentlichen konstant ist und zumindest dem Zeitraum entspricht, der benötigt wird, sodass sich infolge praktisch die gesamte organische Masse in einem geringeren Kristallisationsgrad als die der unerwünschten Abfallstoffe befindet;
- und die Abfallstoffe gleich nach dem Austritt aus dem Bearbeitungsrohr und kurz vor dem Eintritt in die Vergärungsanlage einem Screening-Prozess (9) auf einem Sieb mit Einheitszellengröße L2 unterzogen werden, um eine Stückelung zwischen der organischen Masse mit einem höheren Kristallisationsgrad und den unerwünschten Abfallstoffen sicherzustellen.

2. Verfahren nach Anspruch 1, **gekennzeichnet dadurch, dass** die Aufenthaltszeit (T) des Abfalls im Drehrohr (1) zumindest dem Zeitraum gleich ist, der benötigt wird, sodass sich infolge wenigstens 75 % der aus dem Vorbehandlungsrohr zur Fermentierung austretenden organischen Masse in einem geringeren Kristallisationsgrad als die der unerwünschten Abfallstoffe befindet.

3. Verfahren nach Anspruch 1 oder 2, **gekennzeichnet dadurch, dass** die Abfallzufuhr in das Drehrohr (1) und die Extraktion des behandelten Mülls kontinuierlich stattfindet; der Durchsatz entspricht dem Versorgungsstrom; es geht im Wesentlichen konstant weniger Materie durch Verdampfung innerhalb eines Zeitraumes von einigen Stunden verloren; die Zeitspanne beträgt bevorzugterweise mindestens 10 Stunden und vorteilhaft 24 Stunden.

4. Verfahren gemäß irgendeinem der vorhin angeführten Ansprüche, **gekennzeichnet dadurch, dass** die Einheitszellengröße L1 der Triage zwischen 200 und 600 mm liegt, bevorzugterweise zwischen 350 und 500 mm und vorteilhaft gleichwertig zu 450 mm ist.

5. Verfahren gemäß irgendeinem der vorhin angeführten Ansprüche, **gekennzeichnet dadurch, dass** die Aufenthaltszeit (T) des Abfalls im Drehrohr (1) zwischen zwei und vier Tagen liegt, bevorzugterweise jedoch zwischen zweieinhalb und dreieinhalb Tagen, jedoch vorteilhaft drei Tage beträgt.

6. Verfahren gemäß irgendeinem der vorhin angeführten Ansprüche, **gekennzeichnet dadurch, dass** die Einheitszellengröße (L2) des Siebes vor dem Eintritt in die Vergärungsanlage zwischen 5 und 14 mm beträgt, bevorzugterweise zwischen 7 und 12 mm und vorteilshaft 8mm gleich ist.

7. Verfahren gemäß irgendeinem der vorhin angeführten Ansprüche, **gekennzeichnet dadurch, dass** für die Zufuhr in das Bearbeitungsrohr mindestens ein Dosierbunker (6) eingesetzt wird, um eine reibungslose Glättung der Materie aus abrupten Kristallisationsgrad-Schwankungen und/oder des Volumenstroms des Sammelmülls zu ermöglichen.

8. Verfahren gemäss irgendeinem der vorhin angeführten Ansprüche, **gekennzeichnet dadurch, dass** mehrere aufeinanderfolgende Sortieretagen (10, 16) mit jeweils feineren Einheitszellen jeder nächsten angrenzenden Sortierstufe zwischen dem Ausgang des Drehrohrs (1) und dem letzten Screening-Prozess (9) auf dem Sieb mit Einheitszellengröße L2 vorgesehen sind.

9. Verfahren gemäß irgendeinem der vorhin angeführten Ansprüche, **gekennzeichnet dadurch, dass** der letzte Screening-Prozess vor der Vergärungsanlage (2) durch einen Hackschnitzler mit Trampolineffekt (9) sichergestellt ist.

10. Verfahren gemäss irgendeinem der vorhin angeführten Ansprüche, **gekennzeichnet dadurch, dass** die Vergärungsanlage (2) waagrecht ist, mechanisch funktioniert und eine Fraktion des Gärguts aus der Vergärungsanlage zum Eintritt zurückgeleitet wird, bevorzugterweise gemäß einem Rezirkulationssatz von mindestens 200 %.

11. Verfahren nach Anspruch 10, **gekennzeichnet dadurch, dass** ein Mischbehälter (28) dem letzten Screening-Prozess (9) vorgelagert sowie der Vergärungsanlage (2) nachgelagert ist; ein mehr als 50%iger Anteil der Fraktion des rezirkulierten Gärguts in den Mischbehälter (28) eingeführt wird und der andere Anteil flussab in den Eintritt der Vergärungsanlage.

12. Installation für die Abfallentsorgung für die Durchführung eines Verfahrens gemäß irgendeinem der vorhin angeführten Ansprüche, beinhaltend:
- eine erste Aussortierung durch den Screening-Prozess (4),
- ein Drehrohr (1) für die Vorbehandlung durch Fermentierung der Abfallanteile nach dieser ersten Aussortierung,
- und eine Vergärungsanlage (2), um die aus dem Drehrohr austretende Materie nach Vorbehandlung durch Fermentierung einer Methan-Behandlung zu unterziehen,
insofern gekennzeichnet, dass:
- die Aussortierung des originalen Sammelmülls durch den Screening-Prozess (4) auf einem Sieb mit Einheitszellengröße L1 ohne vorheriges Zerkleinern durchgeführt wird;
- die Transporteinrichtung (6) für die Einführung des Abfalls in das Drehrohr (1) zumindest einen Dosierbunker (6) umfasst, um die reibungslose Glättung der Materie aus abrupten Kristallisationsgrad-Schwankungen und/oder des Volumenstroms des Sammelmülls ohne zeitliche Unterbrechung zu ermöglichen;
- das Drehrohr (1) vorgesehen ist, sodass die Aufenthaltszeit des Abfalls zumindest der Zeit gleich ist, die benötigt wird, damit sich infolge die im wesentlichen gesamte organische Substanz in einem geringeren Kristallisationsgrad als die der unerwünschten Abfallstoffe befindet;
- und stromaufwärts der Vergärungsanlage ein Screening-Verfahren (9) auf einem Sieb mit Einheitszellengröße L2 vorgesehen ist, um eine Stückelung zwischen der organischen Masse mit einem höheren Kristallisationsgrad und den unerwünschten Abfallstoffen sicherzustellen.

13. Installation nach Anspruch 12, **gekennzeichnet dadurch, dass** mehrere Drehrohre (1) vorgesehen sind, die parallel betrieben werden, im Spezielleren drei Drehrohre.

14. Installation nach Anspruch 12 oder 13, **gekennzeichnet dadurch, dass** die Einheitszellengröße L1 des Siebs für die erste Aussortierung zwischen 200 und 600 mm beträgt, bevorzugterweise zwischen 350 und 500 mm und vorteilshaft gleichwertig zu 450 mm ist.

15. Verfahren gemäß irgendeinem der vorhin angeführten Ansprüche von 12 bis 14, **gekennzeichnet dadurch, dass** die Einheitszellengröße L2 des Siebs vor dem Eintritt in die Vergärungsanlage (2) zwischen 5 und 14 mm beträgt, bevorzugterweise zwischen 7 und 12 mm und vorteilshaft gleichwertig zu 8 mm ist.

16. Verfahren gemäß irgendeinem der vorhin angeführten Ansprüche von 12 bis 16, **gekennzeichnet dadurch, dass** es ein Schwingsieb mit Trampolineffekt (9) beinhaltet, das das letzte Screening vor der Vergärungsanlage (2) gewährleistet.

17. Verfahren gemäß irgendeinem der vorhin angeführten Ansprüche von 12 bis 17, **gekennzeichnet dadurch, dass** die Vergärungsanlage (2) waagrecht ist, mechanisch funktioniert und geeignete Mittel vorgesehen sind, um das am Eintritt der Vergärungsanlage austretende Gärgut als Rezirkulationssatz wiederzugewinnen.

## Claims

1. A process for treating waste, in particular household refuse, containing organic matter mixed with undesirable products, especially metals, mineral matter, plastics, and glass, according to which process:
- the waste is subjected to a first sorting by screening (4),
- the fraction of waste passing through the screening is subjected to a prefermentation treatment in a rotating tube (1) with feed at one end and extraction at the other end,
- and the material leaving the prefermentation treatment tube is subjected to a methanization treatment in a digester (2),
**characterized in that**:
- the waste as collected, and without prior crushing, is subjected to the first sorting (4) by screening through screens having a mesh size L1;
- the fraction of waste that has passed through this first sorting operation, which comprises elements of which the large size is less than the value L1, is sent to the prefermentation treatment in the rotating tube (1);
- the rotating tube is fed with waste regularly over time, by smoothing out the fits and starts of collection;
- the residence time (T) of the waste in the rotating tube is substantially constant and at least equal to the time needed so that almost all of the organic matter is in a particle size fraction smaller than that of the undesirable products;
- and the waste, after leaving the rotating tube and before entering the digester, is subjected to a screening (9) through screens having a mesh size L2 that ensures a separation between the organic matter of finer particle size and the undesirable products.

2. The process as claimed in claim 1, **characterized in that** the residence time (T) of the waste in the rotating tube (1) is at least equal to the time needed so that at least 75% by weight of the organic matter leaving the prefermentation tube is in a particle size fraction smaller than that of the undesirable products.

3. The process as claimed in claim 1 or 2, **characterized in that** the feeding of the rotating tube (1) with waste and the extraction of the treated waste are carried out continuously, the extraction flow rate corresponding to the feed flow rate, less the loss of matter and loss by evaporation, and being substantially constant over a duration of several hours, preferably of at least 10 h and advantageously of 24 h.

4. The process as claimed in any one of the preceding claims, **characterized in that** the size L1 of the mesh of the first sorting operation is between 200 and 600 mm, preferably between 350 and 500 mm and advantageously equal to 450 mm.

5. The process as claimed in any one of the preceding claims, **characterized in that** the residence time (T) of the waste in the rotating tube (1) is between two and four days, preferably between two and a half days and three and a half days and advantageously three days.

6. The process as claimed in any one of the preceding claims, **characterized in that** the size (L2) of the mesh of the screen before entry into the digester is between 5 and 14 mm, preferably between 7 and 12 mm and advantageously equal to 8 mm.

7. The process as claimed in any one of the preceding claims, **characterized in that** the feeding of the rotating tube uses at least one metering hopper (6) suitable for smoothing out the possible fits and starts of feeding originating from sudden variations in particle size and/or density of the collected waste stream.

8. The process as claimed in any one of the preceding claims, **characterized in that** several sorting stages (10, 16), with smaller mesh sizes at each following stage, are provided between the outlet of the rotating tube (1) and the last screening (9) by a screen having a mesh size L2.

9. The process as claimed in any one of the preceding claims, **characterized in that** the last screening before the digester (2) is provided by a flip-flow screen (9).

10. The process as claimed in any one of the preceding claims, **characterized in that** the digester (2) is horizontal, mechanically stirred, and a fraction of the digestate leaving the digester is recirculated to the inlet, preferably at a recirculation ratio of at least 200%.

11. The process as claimed in claim 10, **characterized in that** a mixing hopper (28) is provided downstream of the last screening (9) and upstream of the digester (2), and the recirculated digestate fraction is introduced, for a portion of greater than 50%, into the mixing hopper (28), and for the other portion at the inlet of the digester, downstream of the mixing hopper.

12. A waste treatment plant for carrying out a process as claimed in any one of the preceding claims, comprising:
- a first station for sorting by screening (4),
- a rotating tube (1) for the prefermentation treatment of the fraction of waste that has passed through this first sorting operation,
- and a digester (2) in order to subject the material leaving the prefermentation treatment tube to a methanization treatment,
**characterized in that**:
- the first station for sorting by screening (4) has a mesh size L1 for the waste as collected, and without prior crushing;
- means (6) for feeding the rotating tube (1) with waste comprise at least one metering hopper (6) suitable for smoothing out the possible fits and starts of feeding originating from sudden variations in particle size and/or density of the collected waste stream, in order to ensure regular feeding, without interruption over time;
- the rotating tube (1) is provided so that the residence time of the waste is at least equal to the time needed so that almost all of the organic matter is in a particle size fraction smaller than that of the undesirable products;
- and, upstream of the digester, a station for screening (9) through screens having a mesh size L2 is provided in order to ensure a separation between the organic matter of finer particle size and the undesirable products.

13. The plant as claimed in claim 12, **characterized in that** it comprises several rotating tubes (1) operating in parallel, in particular three rotating tubes.

14. The plant as claimed in claim 12 or 13, **characterized in that** the size L1 of the mesh of the screen of the first sorting operation is between 200 and 600 mm, preferably between 350 and 500 mm and advantageously equal to 450 mm.

15. The plant as claimed in any one of claims 12 to 14, **characterized in that** the size L2 of the mesh of the screen before entry into the digester (2) is between 5 and 14 mm, preferably between 7 and 12 mm and advantageously equal to 8 mm.

16. The plant as claimed in any one of claims 12 to 15, **characterized in that** it comprises a flip-flow screen (9) providing the last screening before the digester (2).

17. The plant as claimed in any one of claims 12 to 17, **characterized in that** the digester (2) is horizontal, mechanically stirred, and that means are provided to ensure that a fraction of the outgoing digestate is recirculated to the inlet of the digester.
